# EUROPEAN PATENT APPLICATION

(11) **EP 1 666 603 A1**
(43) Date of publication of application: **07.06.2006**
(21) Application number: 04771245.0
(22) Date of filing: 29.07.2004
(51) Int. Cl.: C12P 21/00, C12N 15/09, G01N 24/08, G01N 33/58, G01N 33/68

(54) **METHOD OF SYNTHESIZING AMINO-ACID-SELECTIVELY LABELED PROTEIN**

(30) Priority: 29.07.2003 JP 2003202926
(71) Applicant: MITSUBISHI CHEMICAL CORPORATION, Tokyo 108-0014 (JP); Endou, Yaeta, Matsuyama-shi, Ehime 791-8016 (JP)
(72) Inventor: ENDOU, Yaeta, Ehime (JP); KOHNO, Toshiyuki MITSUBISHI KAGAKU INST., Machida-shi Tokyo 194-8511 (JP)
(74) Representative: Sternagel, Fleischer, Godemeyer & Partner
(86) International application number: PCT/JP2004/011218
(87) International publication number: WO 2005/010195

(57) **Abstract**

An object of the present invention is to provide a method for synthesizing, in a cell-free protein synthetic system using a wheat germ extract, a protein of interest that is labeled in an amino-acid-selective manner. The present invention provides a method for carrying out a translation reaction of a protein of interest under a condition whereby a metabolic reaction of alanine to aspartic acid and glutamic acid, a metabolic reaction of aspartic acid to glutamic acid or a metabolic reaction of glutamic acid to aspartic acid and/or glutamine is inhibited, while the protein synthesis is not inhibited, when alanine, aspartic acid or glutamic acid of the protein of interest is labeled with a stable isotope in a cell-free protein synthetic system containing a wheat germ extract.

SEQUENCE LISTING
<110> Mitsubishi chemical corporation
<120> Method for producing labeling proteins
<130> A41568A
<160> 2
<170> PatentIn Ver. 2.1
<210> 1
<211> 26
<212> DNA
<213> Artificial Sequence
<220>
<223> Description of Artificial Sequence:synthetic
<400> 1
gggcatatgc aaatttttgt caagac 26
<210> 2
<211> 33
<212> DNA
<213> Artificial Sequence
<220> 33
<223> Description of Artificial Sequence:synthetic
<400> 2
gcggtcgact tagcctcctc tcaatctcaa cac 33

## Description

### TECHNICAL FIELD

The present invention relates to a method for synthesizing a protein of interest wherein amino acids are selectively labeled in a cell-free protein synthetic system using a wheat germ extract. Specifically, the present invention relates to a method for synthesizing a protein wherein alanine, aspartic acid, or glutamic acid is selectively labeled, or the like.

### BACKGROUND ART

A technique of labeling nitrogen atoms in a protein with ¹⁵N, which is a stable isotope capable of being measured by NMR, and then measuring a ¹H·¹⁵N HSQC (heteronuclear single quantum coherence) spectrum, has been widely used for a protein folding test (for example, Montelione, G. T., et al., Nature Struct. Biol., 7, Suppl, 982-985(2000)), determination of a steric structure, ligand screening, or the like.

In general, with regard to preparation of a protein used for the measurement of ¹H·¹⁵N HSQC, using a biosynthetic system such as *Escherichia coli,* yeasts or cultured cells, or using a cell-free synthetic system derived from *Escherichia coli* or wheat germ, a protein of interest is labeled by using an amino acid acting as a nutrient or substrate that has been labeled with ¹⁵N. Thereafter, the protein of interest is purified as necessary, so as to obtain an NMR measurement sample. In the aforementioned ¹H^{·15}N HSQC, amide nitrogen atoms existing in at least the main chains of all, or almost all, amino acids of a protein of interest are generally labeled with ¹⁵N and are used.

On the other hand, the amino-acid-selective labeling method is a method, which comprises synthesizing a protein of interest by labeling one or several types of amino acids contained in the protein of interest with ¹⁵N, ¹³C, D, or a combination thereof, so as to use the protein of interest for NMR measurement. Using this labeling method, information regarding desired amino acids alone can be extracted from the information of the entire protein and can be measured. When a protein wherein all the amino nitrogen atoms of the main chains are labeled with ¹⁵N is subjected to the ¹H·¹⁵N HSQC measurement, signals corresponding to all amino acid residues other than prolines can be obtained. However, when a protein wherein only alanines are labeled with ¹⁵N is produced, for example, and it is then subjected to the ¹H.¹⁵N HSQC measurement, the HSQC signals derived from the alanines alone can be measured. Thus, the division of HSQC signals in terms of amino acid type becomes possible. Moreover, the use of this labeling method with a combination of other labeling methods enables the assignment of signals to specific residues.

Hence, the amino-acid-selective labeling method is used in various ways for NMR measurement. In order to effectively carry out this method, there are several necessary conditions. When a protein of interest is synthesized in large scale using living cells or a cell-free protein synthetic system, this labeling method is carried out by substituting one or several types of substrate amino acids with labeled amino acids. However, if the activity of an amino acid metabolizing enzyme exists in the protein synthetic system, labeled amino acids are converted to other amino acids, and thus a labeling method of interest cannot be carried out. In order to avoid such a situation, it is necessary to construct a protein synthetic system having no amino acid metabolizing enzymes, or to suppress the activity of such an amino acid metabolizing enzymes. However, in a living cell system, it is practically impossible to intend not to contain all amino acid metabolizing enzymes, or to completely suppress the activity thereof. Thus, the analysis of amino acid metabolizing enzymes remaining in a cell extract solution used for various cell-free protein synthetic systems has been attempted. For example, a method of selectively labeling amino acids with ¹⁵N using living *Escherichia coli* cells is as described in Muchmore, D. C., et al, Methods in Enzymology 177, 44-73 (1990) or the like.

The present inventors have already proposed a method, which comprises synthesizing a labeled protein in a cell-free protein synthetic system using a wheat germ extract and then subjecting the obtained protein to the NMR measurement without purification (for example, Japanese Patent Application No. 2003-032381). This method uses a wheat germ extract as among cell-free synthetic systems. Since the types of amino acid metabolizing enzymes remaining in the wheat germ extract have been unknown, it has been impossible to use the above extract for the amino-acid-selective labeling method.

### DISCLOSURE OF THE INVENTION

It is an object of the present invention to provide a method for synthesizing, in a cell-free protein synthetic system using a wheat germ extract, a protein of interest that is labeled in an amino-acid-selective manner.

As a result of intensive studies directed towards the aforementioned object, the present inventors have found that alanine is metabolized to aspartic acid and glutamic acid, aspartic acid is metabolized to glutamic acid, and glutamic acid is metabolized to aspartic acid or glutamine, in a cell-free protein synthetic system containing a wheat germ extract. The inventors have further found that when a metabolism inhibitor for the aforementioned amino acids is added to the above synthetic system, the metabolism of the aforementioned amino acids is inhibited, and amino-acid-selective labeling thereby becomes possible. The present invention has been completed based on these findings.

That is to say, the present invention provides the following:
(1) A method for synthesizing a protein of interest wherein alanine is selectively labeled, which is characterized in that a protein of interest is synthesized in a cell-free protein synthetic system containing a wheat germ extract, under a condition whereby conversion of alanine to aspartic acid and glutamic acid is inhibited while the protein synthesis is not inhibited, by using a substrate wherein alanine has been labeled with a stable isotope.
(2) A method for synthesizing a protein of interest wherein aspartic acid is selectively labeled, which is characterized in that a protein of interest is synthesized in a cell-free protein synthetic system containing a wheat germ extract, under a condition whereby conversion of aspartic acid to glutamic acid is inhibited while the protein synthesis is not inhibited, by using a substrate wherein aspartic acid has been labeled with a stable isotope.
(3) A method for synthesizing a protein of interest wherein glutamic acid is selectively labeled, which is characterized in that a protein of interest is synthesized in a cell-free protein synthetic system containing a wheat germ extract, under a condition whereby conversion of glutamic acid to aspartic acid and/or glutamine is inhibited while the protein synthesis is not inhibited, by using a substrate wherein glutamic acid has been labeled with a stable isotope.
(4) The method according to (1) above, which is characterized in that the condition whereby conversion of alanine to aspartic acid and/or glutamic acid is inhibited while the protein synthesis is not inhibited is that a transaminase inhibitor is present in a concentration range that does not inhibit the protein synthesis.
(5) The method according to (2) above, which is characterized in that the condition whereby conversion of aspartic acid to glutamic acid is inhibited while the protein synthesis is not inhibited is that a transaminase inhibitor is present in a concentration range that does not inhibit the protein synthesis.
(6) The method according to (3) above, which is characterized in that the condition whereby conversion of glutamic acid to aspartic acid and/or glutamine is inhibited while the protein synthesis is not inhibited is that a transaminase inhibitor and a glutamine synthetase inhibitor are present in a concentration range that do not inhibit the protein synthesis.
(7) The method according to any one of (4) to (6) above, which is characterized in that the transaminase inhibitor is aminooxyacetic acid, and the concentration range that does not inhibit the protein synthesis is between 0.01 and 10 mM.
(8) The method according to (6) above, which is characterized in that the glutamine synthetase inhibitor is L-methionine sulfoximine, and the concentration range that does not inhibit the protein synthesis is between 0.01 and 20 mM.
(9) A method for NMR analysis of a protein, which is characterized in that the labeled protein of interest synthesized by the method according to any one of (1) to (8) above is subjected to NMR measurement.
(10) A reagent kit for synthesizing a protein of interest wherein alanine is selectively labeled using a wheat germ extract, which comprises at least a reagent for inhibiting conversion of alanine to aspartic acid and/or glutamic acid.
(11) A reagent kit for synthesizing a protein of interest wherein aspartic acid is selectively labeled using a wheat germ extract, which comprises at least a reagent for inhibiting conversion of aspartic acid to glutamic acid.
(12) A reagent kit for synthesizing a protein of interest wherein glutamic acid is selectively labeled using a wheat germ extract, which comprises at least a reagent for inhibiting conversion of glutamic acid to aspartic acid and/or glutamine.

### BRIEF DESCRIPTION OF THE DRAWINGS

Figure 1 is a view showing the results obtained by measuring the HSQC spectrum of a protein that has been synthesized in a cell-free protein synthetic system containing a wheat germ extract, using a substrate wherein only alanine has been labeled with ¹⁵N.
Figure 2 is a view showing the results obtained by measuring the HSQC spectrum of a protein that has been synthesized in a cell-free protein synthetic system containing a wheat germ extract, using a substrate wherein only aspartic acid has been labeled with ¹⁵N.
Figure 3 is a view showing the results obtained by measuring the HSQC spectrum of a protein that has been synthesized in a cell-free protein synthetic system containing a wheat germ extract, using a substrate wherein only glutamic acid has been labeled with ¹⁵N.
Figure 4 is a graph showing the influence of aminooxyacetic acid concentration upon the ability of a cell-free protein synthetic system containing a wheat germ extract to synthesize a protein.
Figure 5 is a view showing the results obtained by measuring the HSQC spectrum of a protein that has been synthesized in a cell-free protein synthetic system containing a wheat germ extract, using a substrate wherein only alanine or aspartic acid has been labeled with ¹⁵N, in the presence of 1 mM aminooxyacetic acid.
Figure 6 is a view showing the results obtained by measuring the HSQC spectrum of a protein that has been synthesized in a cell-free protein synthetic system containing a wheat germ extract, using a substrate wherein only glutamic acid has been labeled with ¹⁵N, in the presence of 1 mM aminooxyacetic acid.
Figure 7 is a graph showing the influence of L-methionine sulfoxamine concentration upon the ability of a cell-free protein synthetic system containing a wheat germ extract to synthesize a protein.
Figure 8 is a view showing the results obtained by measuring the HSQC spectrum of a protein that has been synthesized in a cell-free protein synthetic system containing a wheat germ extract, using a substrate wherein only glutamic acid has been labeled with ¹⁵N, in the presence of 1 mM aminooxyacetic acid and 0.1 mM L-methionine sulfoxamine.

### BEST MODE FOR CARRYING OUT THE INVENTION

The present invention will be described more in detail below.

### (1) Protein of interest and translation template

Any protein may be used as a protein of interest used in the method of the present invention, as long as several amino acids thereof are selectively labeled by the method of the present invention, and the protein can be synthesized in a cell-free protein synthetic system containing a wheat germ extract. Specific examples of such a protein may include polypeptides, glycoproteins, and their derivatives, covalently-bonded bodies and complexes. As the polypeptide, one comprising 10 to 1,000 amino acid residues is preferably used. In addition, as a glycoprotein, one having a molecular weight between 1,000 and 100,000 is preferable. Specific examples may include a naturally existing protein, a portion thereof, an artificially generated polypeptide, and a protein obtained by adding one or more amino acid residues to the N-terminus or C-terminus of a naturally existing protein. However, examples are not limited thereto. In this case, one or several amino acids may be deleted, substituted, or added, with respect to the amino acid residues of these proteins or polypeptides.

When such a protein is synthesized in a cell-free protein synthetic system containing a wheat germ extract, DNA used as a template is first prepared, and the DNA is then transcribed into RNA. The obtained RNA is provided to the above synthetic system. A preferred example of the DNA used as a template is DNA having a structure whereby a sequence encoding the protein is ligated such that it is under the control of a suitable expression-controlling region. In addition, DNA having a structure whereby a sequence for termination of transcription, a nontranslated region, or the like are ligated downstream thereof, is also preferably used. Such an expression-controlling region includes a promoter, an enhancer, and others.

When SP6 RNA synthetase is used for transcription, an example of a promoter suitable for the cell-free protein synthetic system containing a wheat germ extract is an SP6 promoter. Moreover, it is preferable to insert a nucleotide sequence for enhancing translational activity into a portion between the above promoter sequence and a nucleotide sequence encoding a protein of interest. Specific examples of such a nucleotide sequence for enhancing translational activity in the case of eukaryotes may include: a 5'-cap structure (Shatkin, Cell, 9, 645- (1976)); and a Kozak sequence (Kozak Nucleic Acid. Res., 12, 857 (1984)). Furthermore, it has been found that the 5'-nontranslated leader sequence or the like of an RNA virus also has activity of enhancing translational activity (Japanese Patent No. 2814433). A method for efficiently synthesizing a protein using such sequences has been developed (Japanese Patent Application Laid-Open (Kokai) No. 10-146197). A further example is a sequence obtained by a method for selecting a translation-enhancing sequence using, as an indicator, an "influence" upon the formation of a polysome of a random sequence (Japanese Patent Application No. 2001-396941). Hereinafter, DNA having such a structure may be referred to as "translation template DNA" at times.

An example of a method for preparing translation template DNA is a commonly used known method. Specific examples of such a commonly used known method may include a method using a plasmid vector or a method involving the polymerase chain reaction method. Such methods are described in detail in Sawasaki, T., et al., Proc. Natl. Acad. Sci., 99 (23), 14652- (2002). The thus obtained translation template DNA is transcribed into RNA by a commonly used method, and it is then purified as necessary, so as to obtain mRNA used as a template for a protein of interest used in the method of the present invention.

### (2) Wheat germ extract

In the method of the present invention, a protein of interest is synthesized in a cell-free protein synthetic system containing a wheat germ extract. The type of such a wheat germ extract is not particularly limited, as long as a translation reaction can be carried out by addition of a suitable substrate or the like thereto. Specifically, in the case of a cell-free protein synthetic system containing a wheat germ extract, wheat germ extracts prepared in accordance with publications such as Proc. Natl. Acad. Sci. USA, 97: 559-564 (2000), and Japanese Patent Application Laid-Open (Kokai) Nos. 2000-236896, 2002-125693, and 2002-204689, and cell-free protein synthetic systems containing such wheat germ extracts, can be used.

As a wheat germ extract used in the present invention, a wheat germ extract prepared by separating a germ from albumen in a wheat seed, and extracting and purifying the obtained germ, can be used. Such a wheat germ extract can be prepared from a wheat seed as follows, or a commercially available product can be used. An example of a commercially available cell extract derived from a wheat germ is PROTEIOS™ (manufactured by TOYOBO).

As a method for producing a wheat germ extract, a method described in Johnston, F. B. et al., Nature, 179, 160-161 (1957), or Erickson, A. H. et. Al., (1996) Meth. In Enzymol., 96, 38-50, can be used, for example. The production method will be described more in detail below.

For production of a wheat germ extract used in the present invention, it is preferable that components other than a wheat germ, and particularly; albumen, be first completely eliminated. In order to prepare a germ by such a method, in general, mechanical strength is first added to wheat seeds, so as to obtain a mixture consisting of a germ, an albumen crushed product, and a seed coat crushed product. Thereafter, the albumen crushed production, the seed coat crushed product, and others are eliminated from the above mixture, so as to obtain a crude germ fraction (a mixture containing a germ as a main component and also containing an albumen crushed product and a seed coat crushed product). The strength added to wheat seeds may be strength capable of separating albumen from the wheat seeds. Specifically, plant seeds are crushed using a known crushing device, so as to obtain a mixture consisting of a germ, an albumen crushed product, and a seed coat crushed product.

Wheat seeds can be generally crushed using a known crushing device. It is preferable to use crushing devices that give impact force to a product to be crushed, such as a pin mill or hammer mill. With regard to the degree of crushing, in the case of wheat seeds for example, the seeds are crushed to fragments with a maximum length of generally 4 mm or less, and preferably 2 mm or less. Moreover, such crushing is preferably carried out in a dry system.

Subsequently, from the obtained wheat seed crushed product, a crude germ fraction is obtained using a generally known classifier such as a sieve. For example, in the case of wheat seeds, a crude germ fraction having a mesh size generally between 0.5 and 2.0 mm, and preferably between 0.7 and 1.4 mm, is obtained. Further, seed coats, albumen portions, wastes, or the like contained in the obtained crude germ fraction may be eliminated using wind power or electrostatic power, as necessary.

Moreover, such a crude germ fraction can also be obtained by a method utilizing a difference among a germ, a seed coat, and albumen in terms of specific gravity, such as heavy liquid separation. In order to obtain a crude germ fraction containing a larger amount of germ, the aforementioned methods may be applied in combination. Thereafter, a germ is selected from the obtained crude germ fraction, by visual observation or using a color sorter, for example.

Since there are cases where an albumen component is attached to the thus obtained germ fraction, in general, it is preferable that the germ fraction be further subjected to a washing treatment for purification of the germ. As such a washing treatment, a germ fraction is dispersed and suspended in water or an aqueous solution that has been cooled to generally 10°C or lower, and preferably 4°C or lower, and washing is preferably carried out, until the cloudiness of the washing solution disappears. In addition, it is more preferable that a germ fraction be dispersed and suspended in an aqueous solution containing a surfactant at a temperature of generally 10°C or lower, and preferably 4°C or lower, and that washing be carried out, until the cloudiness of the washing solution disappears. A nonionic surfactant is preferably used herein as a surfactant. A wide range of surfactants can be used, as long as they are nonionic surfactants. Specifically, preferred examples of such a surfactant may include polyoxyethylene derivatives such as Brij, Triton, Nonidet P40, or Tween. Of these, Nonidet P40 is most preferable. Such nonionic surfactants can be used at a concentration of 0.5%, for example. Either a washing treatment with water or an aqueous solution, or a washing treatment with a surfactant, may be carried out. Otherwise, both washing treatments may be carried out. These washing treatments may also be carried out with the combination of an ultrasonic treatment.

In the present invention, a wheat germ is selected from a crushed product obtained by crushing wheat seeds as described above, and the wheat germ is then washed. The thus obtained intact germ (having germinating ability) is fragmented in the presence of an extraction solvent, and the obtained wheat germ extract is separated and further purified, so as to obtain a wheat germ extract used for cell-free protein synthesis.

As such an extraction solvent, an aqueous solution containing a buffer, potassium ions, magnesium ions, and/or a thiol antioxidant, can be used. In addition, calcium ions, L-amino acids, and others may be further added, as necessary. For example, a solution containing N-2-hydroxyethylpiperazin-N'-2-ethanesulfonic acid (HEPES)-KOH, potassium acetate, magnesium acetate, L-amino acid, and/or dithiothreitol, or a solution obtained by modification of the method of Patterson et al. (a solution containing HEPES-KOH, potassium acetate, magnesium acetate, calcium chloride, L-amino acid, and/or dithiothreitol), can be used as an extraction solvent. The composition and concentration of each component contained in such as extraction solvent have already been known, and those used in the production method of a wheat germ extract used for cell-free protein synthesis may be adopted.

A germ is mixed with an extraction solvent with an amount necessary for extraction, and the germ is then fragmented in the presence of the extraction solvent. The amount of such an extraction solvent is generally 0.1 ml or more, preferably 0.5 ml or more, and more preferably 1 ml or more, per gram of the germ before washing. The upper limit of the amount of the extraction solvent is not particularly limited. It is generally 10 ml or less, and preferably 5 ml or less, per gram of the germ before washing. In addition, as a germ to be fragmented, a frozen germ may be used in the conventional way, or an unfrozen germ may also be used. However, an unfrozen germ is more preferably used.

As a fragmentation method, conventionally known crushing methods such as trituration, crushing, impact, or cutting, can be adopted. Fragmentation of the germ by impact or cutting is particularly preferable. The expression "fragmented by impact or cutting" is used herein to mean disruption of a plant germ under conditions whereby the disruption of cell organelle such as a cell nucleus, a mitochondrion or a chloroplast, cell membrane, cell wall, or the like is reduced to a minimum when compared with the conventional trituration or crushing.

The device and method that can be used for fragmentation are not particularly limited, as long as they satisfy the aforementioned conditions. For example, a device having a rapidly rotating edge tool, such as a Waring blender, is preferably used. The number of revolutions of such an edge tool is generally 1,000 rpm or more, and preferably 5,000 rpm or more. It is also generally 30,000 rpm or less, and preferably 25,000 rpm or less. The time of revolution of such an edge tool is generally 5 seconds or more, and preferably 10 seconds or more. The upper limit of the time of revolution is not particularly limited. It is generally 10 minutes or less, and preferably 5 minutes or less. The temperature applied during fragmentation is preferably 10°C or lower, at which operations can be carried out, and particularly preferably approximately 4°C.

Thus, fragmentation of a germ by impact or cutting does not disrupt all the components of the germ such as a cell nucleus or a cell wall, but at least a portion thereof remains without being disrupted. That is to say, since the cell organelle such as a cell nucleus, cell membrane, and cell wall of the germ are not disrupted more than necessary, impurities contained therein, such as DNA or lipids, are mixed to only a slight extent. Thus, RNA, ribosome and the like which are necessary for protein synthesis, and localize in a cytoplasm, can be efficiently extracted from a germ at high purity.

According to this method, the conventional step of crushing a plant germ and a step of mixing the crushed plant germ with an extraction solvent to obtain a wheat germ extract can be carried out simultaneously as a single step. Thus, a wheat germ extract can be efficiently obtained. Hereinafter, the aforementioned method may be referred to as a "blender method" at time.

Subsequently, a wheat germ extract is recovered by centrifugation or the like, and it is then purified by gel filtration or the like, thereby obtaining a wheat germ extract. Such gel filtration can be carried out using a gel filtration device that has previously been equilibrated with a solution (a solvent containing HEPES-KOH, potassium acetate, magnesium acetate, dithiothreitol, or L-amino acid). The composition and concentration of each component contained in a gel filtration solution have already been known. A gel filtration solution used in the production method of a wheat germ extract used for cell-free protein synthesis may be adopted.

There are cases where microorganisms, and particularly the spores of filamentous fungi (molds), are mixed in the germ extract-containing solution after gel filtration. Such microorganisms are preferably removed. In particular, the growth of microorganisms may occur during a cell-free protein synthesis reaction that is performed for a long period of time (1 day or more), and it is important to prevent such growth of microorganisms. A means for removing microorganisms is not particularly limited, but a sterilized filtration filter is preferably used. The pore size of a filter is not particularly limited, as long as it is capable of removing microorganisms that may be mixed. It is generally between 0.1 and 1 micrometer, and preferably between 0.2 and 0.5 micrometers. Since the size of a spore *of Bacillus subtilis,* which is of a relatively small type, is 0.5 µm x 1 µm, it is effective for the removal of such a spore to use a filter of 0.20 micrometers (for example, Minisart™ manufactured by Sartorius). For filtration, it is preferable that filtration be first carried out with a filter of a relatively large pore size, and that filtration be then carried out with a filter of a pore size capable of removing microorganisms that may be mixed.

From the thus obtained cell extract, albumen containing substances suppressing functions to synthesize proteins (substances such as tritin, thionine or ribonuclease, which act on mRNA, tRNA, a protein translation factor, ribosome or the like, so as to suppress the functions thereof) contained or retained in material cells per se, is almost completely removed, and thus the cell extract becomes purified. The expression "albumen is almost completely removed, and thus the cell extract becomes purified" is herein used to mean a wheat germ extract, from which an albumen portion is removed to such an extent that adenine is not substantially removed from ribosome. In addition, the expression "to such an extent that adenine is not substantially removed from ribosome" is used to mean that the adenine removal rate in ribosome is less than 7%, and preferably 1% or less.

Since such a cell extract contains protein synthesis inhibitors having low molecular weights (hereinafter referred to as "low molecular weight synthesis inhibitors" at times), such low molecular weight synthesis inhibitors are fractionated and eliminated from the constitutional components of the cell extract using the difference in molecular weight. It is adequate that the molecular weight of a substance to be eliminated (a low molecular weight inhibitor) be smaller than that of a factor necessary for protein synthesis contained in the cell extract. Specifically, the molecular weight of such a low molecular weight inhibitor is between 50,000 and 14,000, and preferably 14,000 or less.

As a method for eliminating a low molecular weight synthesis inhibitor from a cell extract, commonly used known methods are applied. Specific examples of such a method may include a method involving dialysis through a dialysis membrane, the gel filtration method, and the ultrafiltration method. Of these, the method involving dialysis (dialysis method) is preferable in terms of the easy supply of a substance to an internal dialysis solution. Hereafter, the case of using such a dialysis method will be described in detail.

An example of a dialysis membrane used for dialysis is a membrane having a molecular weight to be eliminated between 50,000 and 12,000. Specifically, a regenerated cellulose membrane having a molecular weight to be eliminated between 12,000 and 14,000 (manufactured by Viskase Sales, Chicago), Spectra/Pore 6 having a molecular weight to be eliminated of 50,000 (manufactured by SPECTRUM LABORATORIES INC., CA, U.S.A.), or the like, is preferably used. A suitable amount of the aforementioned cell extract is placed in such a dialysis membrane, and dialysis is then carried out according to a common method. The time required for such dialysis is preferably between approximately 30 minutes and 24 hours.

When insoluble components are generated in a cell extract during the elimination of low molecular weight synthesis inhibitors, the activity of the finally obtained cell extract (hereinafter referred to as a "treated cell extract" at times) of synthesizing proteins can be increased by inhibiting such generation of insoluble components (hereinafter referred to as "stabilization of a cell extract" at times). A specific example of a method of stabilizing a cell extract is a method of eliminating the low molecular weight inhibitors described in (1) above in a solution containing at least a high-energy phosphate compound such as ATP or GTP. As such a high-energy phosphate compound, ATP is preferably used. In addition, the above method is preferably carried out in a solution, preferably containing ATP and GTP, and more preferably containing ATP, GTP, and 20 types of amino acids.

When low molecular weight inhibitors are eliminated in a solution containing such components (hereinafter referred to as "stabilizing components" at times), such stabilizing components have previously been added to a cell extract, and the mixture is then incubated. Thereafter, the resultant may be subjected to a step of eliminating the low molecular weight inhibitors. When the dialysis method is used to eliminate such low molecular weight synthesis inhibitors, such stabilizing components are added not only to a cell extract, but also to an external dialysis solution, followed by dialysis, thereby eliminating the low molecular weight inhibitors. If such stabilizing components are also added to an external dialysis solution, although the stabilizing components are decomposed during dialysis, fresh stabilizing components are constantly supplied. Thus, this means is favorable. This means can also be applied, when the gel filtration method or the ultrafiltration method is used. Each carrier is equilibrated with a filtration buffer solution containing stabilizing components. Thereafter, a cell extract containing stabilizing components is provided, and filtration is then carried out while the aforementioned buffer solution is added thereto, so that the same above effects can be obtained.

The quantities of such stabilizing components added and the time required for a stabilization treatment can be appropriately selected depending on the type of a cell extract or a preparation method. As a selection method, stabilizing components, the quantities and types of which are determined on a trial basis, are added to a cell extract, and after a suitable period of time, a step of eliminating low molecular weight inhibitors is carried out. Thereafter, the obtained treated cell extract is divided into a soluble component and an insoluble component by methods such as centrifugation. Of these, the condition which gives a small content of insoluble component is selected. Moreover, a method, which comprises conducting cell-free protein synthesis using the obtained treated cell extract and selecting one with high protein synthesizing activity, is also preferable. Furthermore, when a cell extract and the dialysis method are used in the aforementioned selection methods, there is also applied a method, which comprises adding suitable stabilizing components to an external dialysis solution, performing dialysis using such items for a suitable period of time, and then selecting a cell extract depending on the amount of an insoluble component contained in the obtained cell extract or the protein synthesizing activity of the obtained cell extract.

Specifically, when low molecular weight inhibitors are eliminated by the dialysis method using a wheat germ extract prepared by the aforementioned blender method, an example of the thus selected stabilization conditions for the cell extract is conditions whereby 100 µM to 0.5 mM ATP, 25 µM to 1 mM GTP, and 25 µM to 5 mM each of 20 types of amino acids are added to the wheat germ extract and an external dialysis solution, and dialysis is then carried out for 30 minutes to 1 hour or longer. The temperature applied during such dialysis is not particularly limited, as long as the protein synthesizing activity is not lost and dialysis can be carried out at the temperature. Specifically, the minimum temperature is a temperature at which the solution does not freeze, and it is generally -10°C, and preferably -5°C. The maximum temperature is 40°C, which is the limit of a temperature that does not affect a solution used for dialysis, and preferably 38°C.

A method of adding stabilizing components to a cell extract is not particularly limited. Such stabilizing components are added to a cell extract before a step of eliminating low molecular weight inhibitors, and the obtained mixture is then incubated for stabilization for a suitable period of time. Thereafter, a step of eliminating low molecular weight synthesis inhibitors may be carried out. Otherwise, such a step of eliminating low molecular weight synthesis inhibitors may also be carried out, using a cell extract to which stabilizing components have been added, and/or a buffer solution used for the elimination step to which stabilizing components have been added.

As a wheat germ extract used in the present invention, a wheat germ extract having a low content of DNA and/or total fatty acid (palmitic acid, oleic acid, and linolic acid) is preferable. For example, (a) a wheat germ extract having a DNA content of 230 µg/ml or less when an optical density (O.D.) at 260 nm (A260) is 90, or (b) a wheat germ extract having the total amount of total fatty acid (palmitic acid, oleic acid, and linolic acid) of 0.03 g/100 g or less when an optical density (O.D.) at 260 nm (A260) is 90, is preferable. Moreover, a wheat germ extract that satisfies the aforementioned conditions (a) and (b) is particularly preferable.

### (3) Cell-free protein synthesizing reaction

The method of the present invention is a method for synthesizing a protein of interest wherein specific amino acids are selectively labeled with a stable isotope, by translating the protein of interest under conditions whereby amino acids of interest are selectively labeled, in a cell-free protein synthetic system containing the aforementioned wheat germ extract. Herein, in a cell-free protein synthetic system containing a wheat germ extract, as described later in Example 1, alanine is metabolized to aspartic acid and glutamic acid, aspartic acid is metabolized to glutamic acid, and glutamic acid is metabolized to aspartic acid or glutamine, by an amino acid metabolizing enzyme contained in the above extract.

Thus, when the above amino acids are selectively labeled, a protein of interest is synthesized in a cell-free protein synthetic system containing a wheat germ extract under conditions whereby the activity of the above amino acid metabolizing enzyme is inhibited while translation of template RNA into a protein is not inhibited, preferably by using a substrate wherein only the above amino acids are labeled. As a method of analyzing such conditions, first, the concentration of a substance selected as a candidate for inhibiting the above amino acid metabolizing enzyme, which does not inhibit the ability of the above protein synthetic system to synthesize a protein, is determined. For example, suitable protein template RNA is translated with an unlabeled substrate, and a protein obtained after the translation is separated by SDS-polyacrylamide gel electrophoresis and then quantified. Otherwise, an enzyme protein, a method of measuring the activity of which has been known, or a protein having fluorescence, may be translated, and it may be then quantified using the enzyme activity thereof or the fluorescence level thereof as an indicator. By such quantification, the concentration range of an amino acid metabolizing enzyme inhibitor existing in the above protein synthetic system, which does not reduce the amount of a protein synthesized, is determined. Thereafter, a substance inhibiting the metabolism of amino acid of interest is added within the thus determined range of concentration that does not inhibit the translation of template RNA into a protein. For example, template RNA of a protein whose amino acid sequence has been determined is translated, preferably using a substrate wherein only amino acids of interest are labeled with a stable isotope, in the above cell-free protein synthetic system. After completion of the translation, the obtained protein is subjected to NMR measurement described later. Thus, it is confirmed whether other amino acids are not labeled by the substrate added, followed by selection. When the inhibition level of the metabolism of amino acid of interest is changed depending on the concentration of a candidate substance existing in the above synthesis reaction, the concentration sufficient for inhibition of the metabolism of the amino acid is measured. This selection method can be altered using known amino acid metabolizing enzyme inhibitors.

When an alanine-selectively labeled protein is synthesized and when an aspartic acid-selectively labeled protein is synthesized, examples of the thus selected specific conditions include that a transaminase inhibitor is allowed to exist in a translation reaction solution used in a cell-free protein synthesizing method described below within the range that does not inhibit the activity of synthesizing a protein. Herein, transaminase remains in the aforementioned wheat germ extract and has the activity of metabolizing alanine to aspartic acid and glutamic acid and/or the activity of metabolizing aspartic acid to glutamic acid. As a transaminase inhibitor for inhibiting such transaminase activity, an inhibitor whose concentration range that inhibits transaminase activity and whose concentration range that does not inhibit the synthesis of a protein of interest are overlapped in the above synthetic system is preferably used. A specific example of such a trasaminase inhibitor is aminooxyacetic acid. The concentration thereof is preferably between 0.01 and 10 mM.

Moreover, examples of conditions applied in the case of synthesizing a glutamic acid-selectively labeled protein include that a transaminase inhibitor and a glutamine synthetase inhibitor are allowed to exist in a translation reaction solution used in a cell-free protein synthesizing method described below within the concentration range that does not inhibit the synthesis of a protein. Herein, transaminase remains in the aforementioned wheat germ extract and has the activity of metabolizing glutamic acid to aspartic acid, whereas glutamine synthetase remains in the aforementioned wheat germ extract and has the activity of metabolizing glutamic acid to glutamine. As a transaminase inhibitor for inhibiting such transaminase activity or a glutamine synthetase inhibitor, an inhibitor whose concentration range that inhibits the transaminase activity or glutamine synthetase activity and whose concentration range that does not inhibit the synthesis of a protein of interest are overlapped in the above synthetic system is preferably used. A specific example of such a trasaminase inhibitor is aminooxyacetic acid. The concentration thereof is preferably between 0.01 and 10 mM. In addition, a specific example of such a glutamine synthetase inhibitor is L-methionine sulfoximine. The concentration thereof is preferably between 0.01 and 20 mM.

The type of a cell-free protein synthesis method applied under such conditions is not particularly limited, as long as it is a method comprising adding template RNA, a substrate, an energy source, etc. to the aforementioned wheat germ extract and further adding thereto the aforementioned substance necessary for inhibiting amino acid metabolic activity, so as to synthesize a protein of interest. A synthesis reaction solution comprises the aforementioned cell extract, template RNA amino acid acting as a substrate, specific amino acids labeled with stable isotopes such as ¹⁵N, ¹³C, or ²H, an energy source, various types of ions, a buffer solution, an ATP-regenerating system, a nuclease inhibitor, tRNA, a reducing agent, polyethylene glycol, 3',5'-cAMP, folate, an antibacterial agent, and others. These substances are selected as appropriate depending on the type of a protein of interest, and are then prepared.

Amino acids acting as a substrate are 20 types of amino acids constituting a protein, wherein at least alanine, aspartic acid, or glutamic acid is specifically labeled with a stable isotope. The method of the present invention involves selective labeling of alanine, aspartic acid, or glutamic acid. Thus, substrate amino acid used during the synthesis of a protein of interest may be labeled or may not be labeled, except for amino acids to be selectively labeled. However, labeling in this case differs from the labeling of amino acids to be selectively labeled. The concentration of such a substrate is suitably between 0.05 and 0.4 mM. In addition, examples of an energy source may include ATP and GTP. ATP is preferably added at a concentration between 1.0 and 1.5 mM, and GTP is added at a concentration between 0.2 and 0.3 mM. With regard to various types of ions and a suitable concentration thereof in a reaction solution, 60 to 120 mM potassium acetate, 1 to 10 mM magnesium acetate, etc. are preferable. As a buffer solution, 15 to 35 mM Hepes-KOH, 10 to 50 mM Tris-acetate, or the like is used. Moreover, as an ATP-regenerating system, a combination of phosphoenol pyruvate with pyruvate kinase, or a combination of 12 to 20 mM creatine phosphate with 0.2 to 1.6 µg/µl creatine kinase, is used. As a nuclease inhibitor, a 0.3 to 3.0 U ribonuclease inhibitor or a 0.3 to 3 U nuclease inhibitor is used per µl of a reaction solution.

Of these, a specific example of a ribonuclease inhibitor is human placenta-derived RNase inhibitor (manufactured by TOYOBO). Further, tRNA can be obtained by the method described in R., et al., Biochim. Biophys. Acta., 43, 1 (1960) or the like, or a commercially available product can also be used. An example of a reducing agent is 0.1 to 3.0 mM dithiothreitol. Examples of an antibacterial agent may include 0.001% to 0.0 1 % sodium azide and 0.1 to 0.2 mg/ml ampicillin. An example of a nucleic acid stabilizer used herein is 0.3 to 0.5 mM spermidine.

A culture temperature is between 10°C and 40°C, preferably between 15°C and 30°C, and more preferably between 20°C and 26°C. A reaction time is not particularly limited, as long as it enables protein synthesis. When a system for supplying a substance that is consumed during a translation reaction is used, as in the present invention, the reaction is maintained for 24 to 75 hours.

Examples of a system or device for protein synthesis may include: a method involving addition of an energy source, amino acid, or tRNA that are necessary for cell-free protein synthesis, to the above cell extract, such as the batch method (Pratt, J. M. et al.,Transcription and Tranlation, Hames, 179-209, B. D. & Higgins, S. J., eds, IRL Press, Oxford (1984)); a continuous cell-free protein synthetic system for continuously supplying amino acid, an energy source, or the like, to a reaction system (Spirin, A. S. et al., Science, 242, 1162-1164 (1988)); the dialysis method (Kikawa et al., the 21^{st} Annual Meeting of the Molecular Biology Society of Japan, WID6); and the double layer method (Sawasaki, T., et al., FEBS Let. ,514, 102-105 (2002)). Furthermore, a method of supplying template RNA, amino acid, an energy source, or the like to a synthesis reaction system when necessary, and eliminating a synthetic product or decomposed product when necessary (Japanese Patent Application Laid-Open (Kokai) No. 2000-333673; hereinafter referred to as a "discontinuous gel filtration method" at times), or other methods, can also be used.

### (4) Recovery and purification of protein of interest

The thus synthesized protein of interest wherein specific amino acids are labeled with a stable isotope is recovered from the reaction solution, and if necessary, the protein of interest is purified by an appropriate method, thereby obtaining a protein of interest. However, when a protein of interest is used for NMR measurement, purification is not always necessary. It is sufficient in many cases that the protein be concentrated to a suitable concentration by a known method, and that the buffer solution be exchanged with a fresh buffer solution used for the NMR measurement. An example of such a concentration method is a method using an ultrafiltration concentration device. In addition, in order to exchange the buffer solution, a method using a commercially available spin column is preferably applied.

### (5) NMR measurement

When the thus synthesized protein of interest wherein specific amino acids are labeled with ¹⁵N, ¹³C, D, or a stable isotope thereof is used for the NMR measurement, information regarding desired amino acids alone can be extracted from the entire protein information, and it can be then measured. As an NMR measurement method used herein, any method can be applied, as long as it can be used for NMR, regardless of solution or solid. Specifically, either the homogeneous nuclear multidimensional NMR measurement method or the heterogeneous nuclear multidimensional NMR measurement method may be applied. Examples of a solution homogeneous nuclear multidimensional NMR measurement method may include COSY, TOCSY, NOESY, and ROESY [Cavanagh, W. J. et al., Protein NMR Spectroscopy, Principles and Practice, Academic Press (1996)]. Examples of the heterogeneous nuclear multidimensional NMR measurement method may include measurement methods such as HSQC, HMQC, CH-COSY, CBCANH, CBCA(CO)NH, HNCO, HN(CA)CO, HNHA, H(CACO)NH, HCACO, 15N-edited NOESY-HSQC, 13C-edited NOESY-HSQC, 13C/15N-edited HMQC-NOESY-HMQC, 13C/13C-edited HMQC-NOESY-HMQC, 15N/15N-edited HSQC-NOESY-HSQC [Cavanagh, W. J., et al., Protein NMR Spectroscopy. Principles and Practice, Academic Press (1996)], HN(CO)CACB, HN(CA)CB, HN(COCA)CB [Yamazaki, T., et al., J. Am. Chem. Soc., 116 (1994) 11655-11666], H(CCO)NH, C(CO)NH [Grzesiek, S., et al., J. Magn. Reson., B 101 (1993) 114-119], CRIPT, CRINEPT [Riek, R., et al., Proc. Natl. Acad. Sci. USA., 96 (1999) 4918-4923], HMBC, HBHA(CBCACO)NH [Evans J. N. S., Biomolecular NMR Spectroscopy. Oxford University Press (1995) 71], INEPT [Morris, G. A., et al., J. Am. Chem. Soc., 101 (1979) 760-762], HNCACB [Wittekind, M., et al., J. Magn. Reson. B 101 (1993) 201], HN(CO)HB [Grzesiek, S., et al., J. Magn. Reson. 96 (1992) 215-222.], HNHB [Archer, S. J., et al., J. Magn. Reson., 95 (1991) 636-641], HBHA(CBCA)NH [Wang, A.C., et al., J. Magn. Reson., B 105 (1994) 196-198.], HN(CA)HA [Kay, L.E., et al., J. Magn. Reson., 98 (1992)443-450], HCCH-TOCSY [Bax, A., et al., J. Magn. Reson., 88 (1990) 425-431], TROSY [Pervushin, K., et al., Proc. Natl. Acad. Sci., 94 (1997) 12366-12371], 13C /15N-edited HMQC-NOESY-HSQC [Jerala R, et al., J. Magn. Reson., 108 (1995) 294-298], HN(CA)NH [Ikegami, T., et al., J. Magn. Reson., 124 (1997) 214217], and HN(COCA)NH [Grzesiek, S., et al., J. Biomol. NMR, 3 (1993) 627-638.]. However, examples are not limited thereto.

### (6) Labeled protein synthesizing kit

The kit of the present invention includes at least a reagent for inhibiting the change of alanine to aspartic acid and/or glutamic acid, which is used to synthesize an alanine-selectively labeled protein of interest using a wheat germ extract. In addition, the present kit includes at least a reagent for inhibiting the change of aspartic acid to glutamic acid, which is used to synthesize an aspartic acid-selectively labeled protein of interest using a wheat germ extract. Moreover, the present kit includes at least a reagent for inhibiting the change of glutamic acid to aspartic acid and/or glutamine, which is used to synthesize a glutamic acid-selectively labeled protein of interest using a wheat germ extract.

Using the above kit, a protein of interest wherein specific amino acids are labeled can be simply and easily synthesized using a wheat germ extract.

The above kit may adopt any structure, as long as it enables the synthesis of the protein of interest of the present invention. Specifically, if it is a kit for synthesizing an alanine-selectively labeled protein of interest, it comprises at least a reagent for inhibiting the change of alanine to aspartic acid and/or glutamic acid, and it may further comprise a wheat germ extract, an enzyme and a buffer solution that are used for synthesis, and labeled or unlabeled amino acid. As such reagents, those as described above are used, or they can also be produced by the combined use of known reagents.

### EXAMPLES

The present invention will be described in detail in the following examples. However, these examples are not intended to limit the scope of the present invention.

### Example 1 NMR measurement of protein of interest synthesized using substrate wherein only one type of amino acid has been labeled

### (1) Preparation of template mRNA

The yeast ubiquitin gene (Genbank Accession No. X01474) was amplified from *Saccharomyces cerevisiase* strain by the PCR method using, as a template, yeast genomic DNA prepared by the Hereford method (L. Hereford, et al., Cell 18, 1261-1271 (1979)), and using primers having the nucleotide sequences shown in SEQ ID NOS: 1 and 2. The amplified product was then introduced into the SpeI-SalI site of the plasmid pEU3b (Sawasaki, T., Proc. Natl. Acad. Sci. USA., 99(23), 14652-14657 (2002). In the presence of 16 mM magnesium ions, using the above plasmid as a template, yeast ubiquitin mRNA was transcribed using SP6 RNA polymerase (manufactured by Promega) and synthesized. In addition, using pEU-GFP (Sawasaki, T., Proc. Natl. Acad. Sci. USA., 99(23), 14652-14657 (2002) as a template, GFP mRNA was transcribed using SP6 RNA polymerase (manufactured by Promega) and synthesized.

### (2) Synthesis of protein of interest using substrate wherein only one type of amino acid has been labeled

The mRNA synthesized in the aforementioned Example 1 was concentrated to result in a concentration of 100 µg/130 µl, and it was then mixed with a wheat germ extract (Proteios™ , manufactured by TOYOBO) (1 ml). The mixed solution was allowed to react for 2 days with a dialysis buffer solution, wherein only one type of amino acid had been labeled with ¹⁵N (manufactured by Cambridge Isotope Laboratories) and 19 types of remaining amino acids were ordinary amino acids. Thereafter, the dialysis buffer solution was exchanged with a fresh buffer solution, and the protein synthesis reaction was carried out further for 2 days. Thereafter, 1 ml of the reaction solution was concentrated to 250 µl using a Centricon-3 ultrafiltration concentration device manufactured by Millipore. The concentration of the yeast ubiquitin protein in the concentrated solution was found to be 120 µM. The concentrated solution was passed through a Micro Spin G-25 gel filtration column manufactured by Amersham that had previously been equilibrated with a buffer solution used for NMR measurement (50 mM sodium phosphate, pH 6.5, 100 mM NaCl), so that the buffer solution was exchanged with a buffer solution used for the measurement, thereby preparing an NMR measurement sample.

### (3) NMR measurement

For the NMR measurement, an Avance-500 spectrometer manufactured by Bruker was used. In order to maintain the stability of a magnetic field, 10% D₂O used for NMR lock was added to the measurement sample, and thereafter, ¹H-¹⁵N HSQC measurement was carried out. The measurement temperature was set at 30°C.

Yeast ubiquitin proteins were synthesized such that only one type of amino acid was labeled with ¹⁵N and the other amino acids were not labeled. The HSQC spectra of such proteins were then measured. As a result, it was found that with regard to ¹⁵N-labeled amino acids other than alanine, aspartic acid, and glutamic acid, the metabolism of the amino acids did not take place, and that only amino acids, into which ¹⁵N labeling had been introduced, could be selectively labeled.

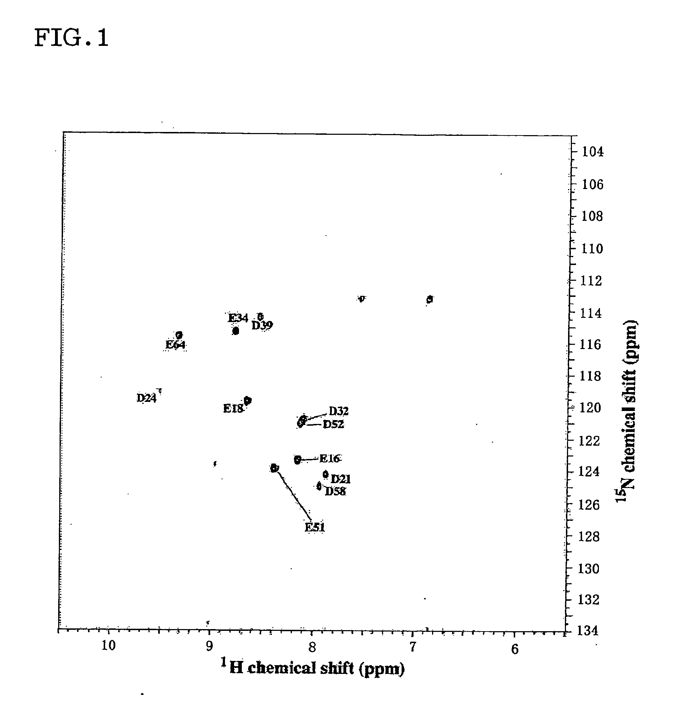

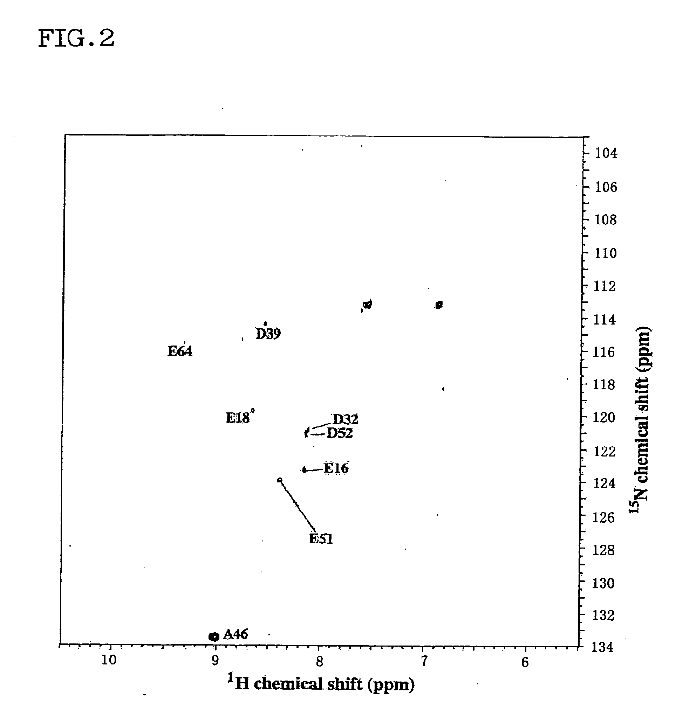

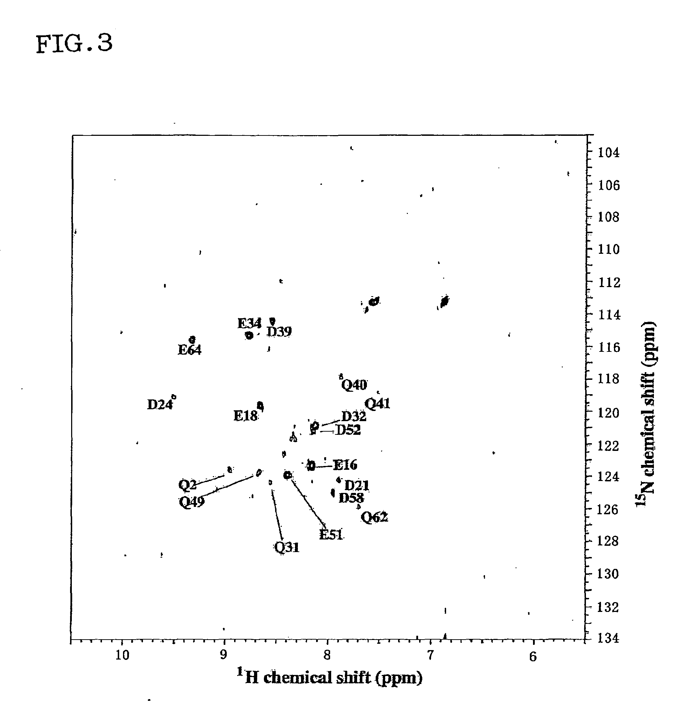

However, in the case of using amino acids wherein only alanine had been labeled with ¹⁵N, not only the alanine signal but also HSQC signals derived from aspartic acid and glutamic acid were observed (Figure 1). In addition, in the case of using amino acids wherein only aspartic acid had been labeled with ¹⁵N, not only the aspartic acid signal but also an HSQC signal derived from glutamic acid was observed (Figure 2). Moreover, in the case of using amino acids wherein only glutamic acid had been labeled with ¹⁵N, not only the glutamic acid signal but also HSQC signals derived from aspartic acid and glutamine were observed (Figure 3).

### Example 2 Studies regarding influence by addition of transaminase inhibitor upon amino acid metabolism and ability to synthesize protein

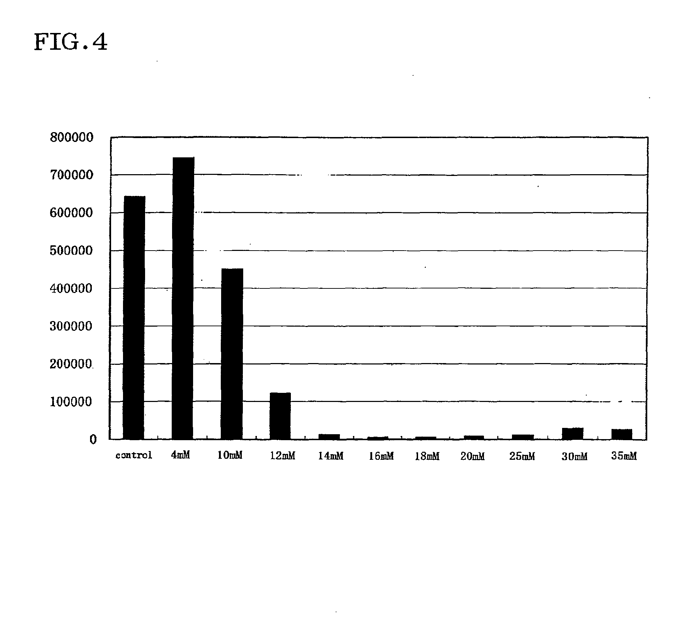

Among activities of amino acid metabolizing enzymes existing in the cell-free protein synthetic system containing a wheat germ extract observed in Example 1, the influence by the inhibition of transaminase activity upon the aforementioned amino acid metabolism and the activity of synthesizing a protein was examined. When a protein of interest was synthesized by the same method as described in Example 1, the concentration of aminooxyacetic acid acting as a transaminase inhibitor was set at each of 4, 10, 12, 14, 16, 18, 20, 25, 30, and 35 mM in an external dialysis solution and a reaction solution. Thereafter, the amount of GFP synthesized by wheat germ protein synthesis was examined using the fluorescence intensity of the GFP as an indicator. As shown in Figure 4, it was found that the amount of GFP synthesized was not reduced under conditions whereby the concentration of aminooxyacetic acid was 4 mM or less, and that even in the concentration of 10 mM, approximately 70% of the synthesized amount thereof was maintained.

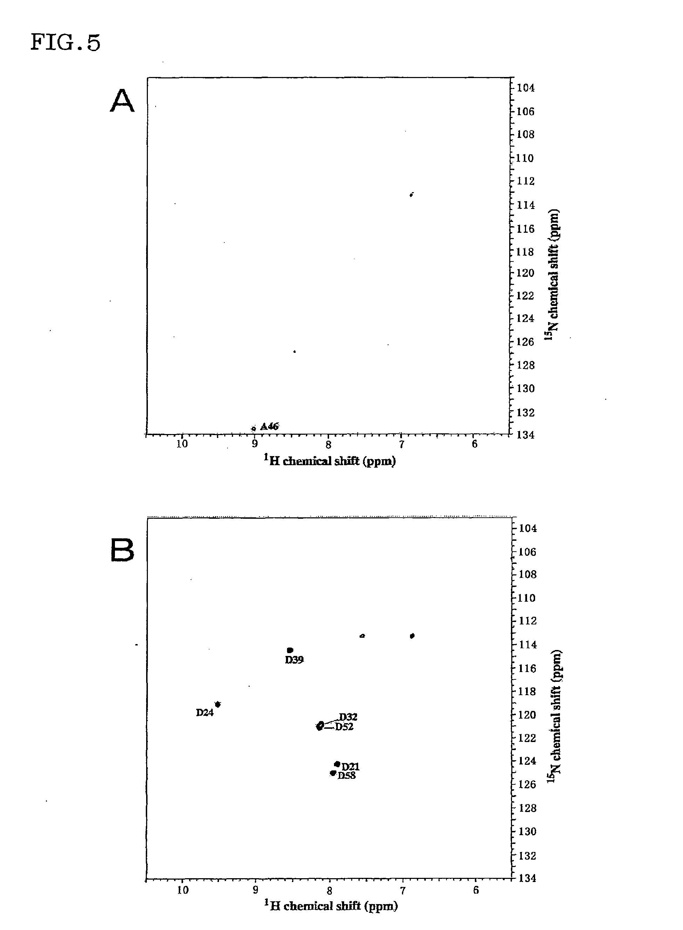

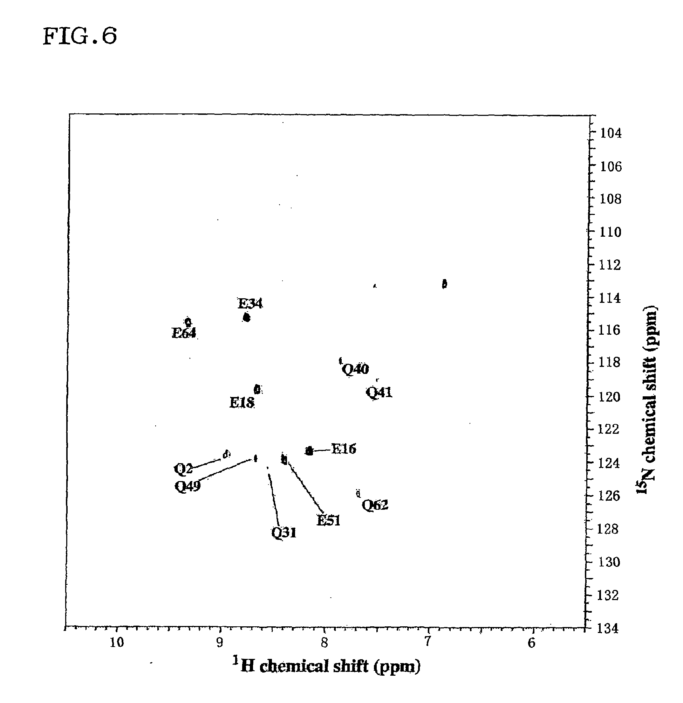

Subsequently, the concentration of aminooxyacetic acid in a translation solution and an external dialysis solution was set at 1 mM. The following 3 types of substrate amino acids were prepared: (1) only alanine was labeled with ¹⁵N and 19 types of remaining amino acids were ordinary amino acids; (2) only aspartic acid was labeled with ¹⁵N and 19 types of remaining amino acids were ordinary amino acids; and (3) only glutamic acid was labeled with ¹⁵N and 19 types of remaining amino acids were ordinary amino acids. Using these substrate amino acids, yeast ubiquitins were synthesized by the same method as described in Example 1, and the ¹H-¹⁵N HSQC was measured.
Regarding (1) and (2) above, only signal derived from alanine or aspartic acid was observed (Figure 5A (alanine) and Figure 5B (aspartic acid)), and thus it was confirmed that selective labeling became possible. Regarding (3) above, it could be confirmed that the metabolism of glutamic acid to aspartic acid was suppressed. However, at the same time, it was also confirmed that the activity of metabolizing glutamic acid to glutamine has still remained (Figure 6). Moreover, the aforementioned experiment was carried out under conditions whereby the concentration of aminooxyacetic acid was set at 0.1 µM, 1 µM, 10 µM, 100 µM, and 1 mM. As a result, it was found that two thirds of the metabolism was inhibited by aminooxyacetic acid with a concentration of 10 µM, and that the metabolism was completely inhibited by aminooxyacetic acid with a concentration of 100 µM or more.

### Example 3 Studies regarding influence by addition of glutamine synthetase inhibitor upon amino acid metabolism and ability to synthesize protein

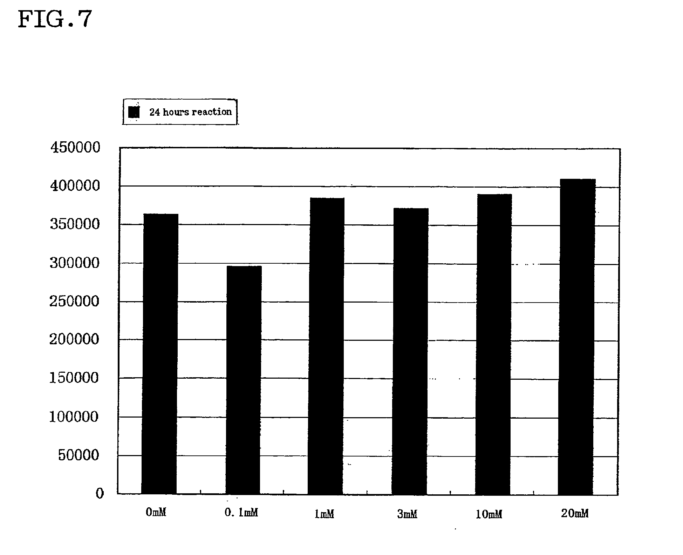

The activity of an amino acid metabolizing enzyme to metabolize glutamic acid to glutamine in the cell-free protein synthetic system containing a wheat germ extract was observed in Example 2. The influence by such inhibition upon the aforementioned amino acid metabolism and the activity of synthesizing a protein was examined. When a protein of interest was synthesized by the same method as described in Example 1, the concentration of L-methionine sulfoximine acting as a glutamine synthetase inhibitor was set at each of 0, 0.1, 1, 3, 10, and 20 mM in an external dialysis solution and a reaction solution. Thereafter, the amount of GFP synthesized by wheat germ protein synthesis was examined using the fluorescence intensity of the GFP as an indicator. As shown in Figure 7, it was found that the amount of GFP synthesized was not reduced under conditions whereby the concentration of L-methionine sulfoximine was 20 mM or less.

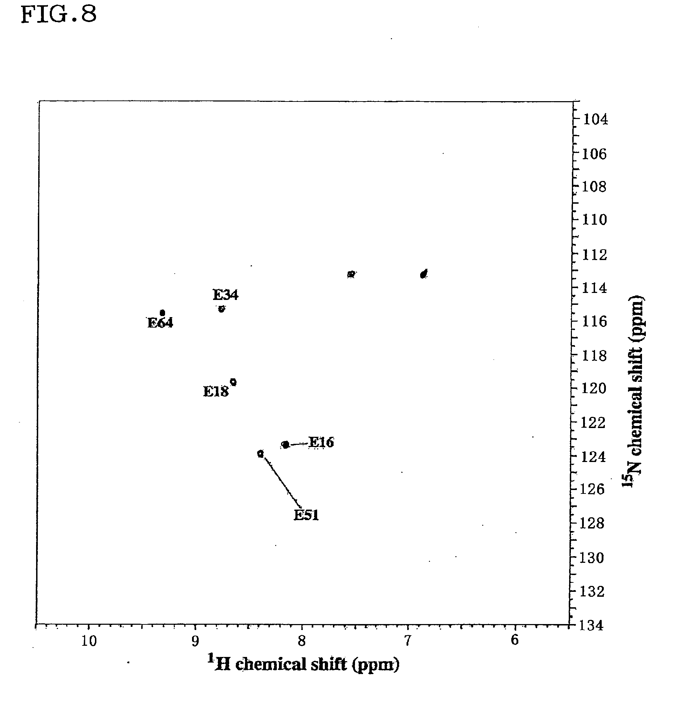

Subsequently, the concentration of aminooxyacetic acid was set at 1 mM and the concentration of L-methionine sulfoximine was set at 0.1 mM in a translation solution and an external dialysis solution. Substrate amino acid was prepared such that only glutamic acid was labeled with ¹⁵N and 19 types of remaining amino acids were ordinary amino acids. Using such substrate amino acid, yeast ubiquitin was synthesized by the same method as described in examples, and the ¹H-¹⁵N HSQC was measured. As a result, it could be confirmed that both the metabolism of glutamic acid to aspartic acid and the metabolism of glutamic acid to glutamine were suppressed (Figure 8), and it was found that glutamic acid was selectively labeled with ¹⁵N. Moreover, the aforementioned experiment was carried out under conditions whereby the concentration of L-methionine sulfoximine was set at 0.1 µM, 1 µM, 10 µM, and 100 µM. As a result, it was found that two thirds of the metabolism was inhibited by L-methionine sulfoximine with a concentration of 10 µM, and that the metabolism was completely inhibited by L-methionine sulfoximine with a concentration of 100 µM or more.

### INDUSTRIAL APPLICABILITY

The method of the present invention enables the synthesis of a protein of interest wherein all amino acids are selectively labeled, in a cell-free protein synthetic system containing a wheat germ extract. Since the protein synthesized in such a cell-free protein synthetic system containing a wheat germ extract can be subjected to NMR measurement without purification thereof, the method of the present invention provides a method for more simply and easily measuring a protein based on the NMR measurement.

The present application claims priority from Japanese Patent Application No. 2003-202926, filed on July 29, 2003; the disclosure of which is hereby incorporated by reference. In addition, all publications cited in the present specification are incorporated herein by reference in their entirety.

## Claims

1. A method for synthesizing a protein of interest wherein alanine is selectively labeled, which is **characterized in that** a protein of interest is synthesized in a cell-free protein synthetic system containing a wheat germ extract, under a condition whereby conversion of alanine to aspartic acid and glutamic acid is inhibited while the protein synthesis is not inhibited, by using a substrate wherein alanine has been labeled with a stable isotope.

2. A method for synthesizing a protein of interest wherein aspartic acid is selectively labeled, which is **characterized in that** a protein of interest is synthesized in a cell-free protein synthetic system containing a wheat germ extract, under a condition whereby conversion of aspartic acid to glutamic acid is inhibited while the protein synthesis is not inhibited, by using a substrate wherein aspartic acid has been labeled with a stable isotope.

3. A method for synthesizing a protein of interest wherein glutamic acid is selectively labeled, which is **characterized in that** a protein of interest is synthesized in a cell-free protein synthetic system containing a wheat germ extract, under a condition whereby conversion of glutamic acid to aspartic acid and/or glutamine is inhibited while the protein synthesis is not inhibited, by using a substrate wherein glutamic acid has been labeled with a stable isotope.

4. The method according to claim 1, which is **characterized in that** the condition whereby conversion of alanine to aspartic acid and/or glutamic acid is inhibited while the protein synthesis is not inhibited is that a transaminase inhibitor is present in a concentration range that does not inhibit the protein synthesis.

5. The method according to claim 2, which is **characterized in that** the condition whereby conversion of aspartic acid to glutamic acid is inhibited while the protein synthesis is not inhibited is that a transaminase inhibitor is present in a concentration range that does not inhibit the protein synthesis.

6. The method according to claim 3, which is **characterized in that** the condition whereby conversion of glutamic acid to aspartic acid and/or glutamine is inhibited while the protein synthesis is not inhibited is that a transaminase inhibitor and a glutamine synthetase inhibitor are present in a concentration range that do not inhibit the protein synthesis.

7. The method according to any one of claims 4 to 6, which is **characterized in that** the transaminase inhibitor is aminooxyacetic acid, and the concentration range that does not inhibit the protein synthesis is between 0.01 and 10 mM.

8. The method according to claim 6, which is **characterized in that** the glutamine synthetase inhibitor is L-methionine sulfoximine, and the concentration range that does not inhibit the protein synthesis is between 0.01 and 20 mM.

9. A method for NMR analysis of a protein, which is **characterized in that** the labeled protein of interest synthesized by the method according to any one of claims 1 to 8 is subjected to NMR measurement.

10. A reagent kit for synthesizing a protein of interest wherein alanine is selectively labeled using a wheat germ extract, which comprises at least a reagent for inhibiting conversion of alanine to aspartic acid and/or glutamic acid.

11. A reagent kit for synthesizing a protein of interest wherein aspartic acid is selectively labeled using a wheat germ extract, which comprises at least a reagent for inhibiting conversion of aspartic acid to glutamic acid.

12. A reagent kit for synthesizing a protein of interest wherein glutamic acid is selectively labeled using a wheat germ extract, which comprises at least a reagent for inhibiting conversion of glutamic acid to aspartic acid and/or glutamine.
